# EUROPÄISCHE PATENTANMELDUNG

(11) **EP 4 710 952 A1**
(43) Veröffentlichungstag der Anmeldung: **18.03.2026**
(21) Anmeldenummer: 24315418.4
(22) Anmeldetag: 16.09.2024
(51) Int. Cl.: A61L 15/22, A61L 15/60

(54) **WUNDAUFLAGE ZUR WUNDBEHANDIUNG IM FEUCHTEN ODER FEUCHT-NASSEN MILIEU**

(71) Anmelder: Paul Hartmann AG, 89522 Heidenheim (DE)
(72) Erfinder: KETTEL, Markus, 29522 Heidenheim (DE); KARL, Michael Maximilian, 89522 Heidenheim (DE); MERCKEL, Fabien, 67100 Strasbourg (FR); VRANA, Nihal Engin, 67100 Strasbourg (FR); HATHROUBI, Skander, 67100 Strasbourg (FR)
(74) Vertreter: Paul Hartmann AG Patents & Licensing

(57) **Zusammenfassung**

Die Erfindung betrifft eine Wundauflage (2) zur Wundbehandlung im feuchten oder feucht-nassen Milieu, mit einem faservliesbasierten Saug-/Spülkörper (4), in dem superabsorbierendes Material verteilt aufgenommen ist, wobei der Saug-/Spülkörper (4) herstellerseitig mit einer salzhaltigen wässrigen Lösung, insbesondere Ringerlösung beaufschlagt ist, und mit einer die äu-βeren Sichtseiten der Wundauflage bildenden Umhüllung (6), wobei die Umhüllung (6) auf der wundzugewandten Seite der Wundauflage ein textiles Flächenmaterial (9), insbesondere aus einem Gestrick, Gewirke oder Gewebe, umfasst, wobei die Umhüllung (6) auf der wundzugewandten Seite der Wundauflage eine außenseitig teilweise oder vollständig aufgebrachte antimikrobielle Beschichtung (22) aufweist, welche eine Hyaluronsäure sowie ein Polypeptid ausgewählt aus Polyarginin, Polylysin und Polyornithin oder eine Mischung aus wenigstens zwei der zuvor genannten Polypeptide umfasst.

## Beschreibung

Die Erfindung betrifft eine Wundauflage zur Wundbehandlung im feuchten oder feuchtnassen Milieu, mit einem faservliesbasierten Saug-/Spülkörper, in dem superabsorbierendes Material verteilt aufgenommen ist, wobei der Saug-/Spülkörper herstellerseitig mit einer salzhaltigen wässrigen Lösung, insbesondere Ringerlösung beaufschlagt ist. Eine die äußeren Sichtseiten der Wundauflage bildende Umhüllung umfasst auf der wundzugewandten Seite der Wundauflage eine Schicht aus einem textilen Flächenmaterial, insbesondere aus einem Gestrick, Gewirke oder Gewebe.

Eine derartige Wundauflage ist aus WO 2011/141454 A1 der Anmelderin bekannt. Es handelt sich hierbei um eine wundkissenartige oder kompressenartige Wundauflage, die auf eine Wunde aufbringbar ist oder auch zum Austamponieren tiefer Wunden verwendet werden kann. Der Saug-/Spülkörper wird herstellerseitig, insbesondere bis zur Sättigung mit einer salzhaltigen wässrigen Lösung beaufschlagt, welche das superabsorbierende Material quellen und in einen gelartigen' Zustand übergehen lässt. Dies verleiht dem Saug-/Spülkörper eine duale Funktion bei Wunden mit starker Exsudation. Wundsekrete werden einschließlich ihrer kritischen Bestandteile wie Keime durch den Saug-/Spülkörper aktiv aufgenommen und darin gehalten, wobei der Saug-/Spülkörper im Gegenzug die salzhaltige wässrige Lösung an die Wunde abgibt und somit ein feuchtes Wundmilieu schafft oder unterstützt. Hierdurch wird die Wundreinigung und eine positive Wundkonditionierung unterstützt und somit die Heilung positiv beeinflusst. Man spricht hier von interaktiver Nasstherapie, die insbesondere bei schlecht heilenden Wunden, bei klinisch manifest infizierten Wunden oder bei chronischen Wunden mit unterschiedlicher Genese, wie diabetisches Gangrän, Dekubitalulcus oder Ulcus cruris bevorzugt Anwendung findet.

Bei der vorerwähnten Ringerlösung handelt es sich typischerweise um eine wässrige Lösung mit Natriumchlorid, Kaliumchlorid und Calciumchlorid (insbesondere 8,6 g NaCl, 0,3 g KCl und 0,33 g CaCl₂ je Liter).

Des Weiteren sind Wundauflagen mit antibiotischer Wirksamkeit bekannt. Diese beruhen in der Regel auf der Verwendung körperfremder antimikrobieller Wirkstoffe. Derartige Wundauflagen zeigen zwar eine Wirkung gegen pathogene Mikroorganismen, haben jedoch die nachteilige Eigenschaft auch körpereigene Zellen zu beeinträchtigen. Mittels in vitro Assays konnte eine erhöhte Zytotoxizität für tierische Zellen nachgewiesen werden. Die betroffenen Zellen werden gestresst und ihre Vitalwerte sinken ab. Bei Testkonzentrationen, die den Realbedingungen nahekommen, stirbt für gewöhnlich ein Teil der im Assay befindlichen Zellen ab.

Die EP 2 371 335 B1 beschreibt eine antibakterielle Wundauflage mit dem Wirkstoff Polyhexamethylenbiguanid (PHMB). Jüngste Untersuchungen haben jedoch gezeigt, dass PHMB toxische Effekt im in vitro Assay bei Humanzellen verursacht (Medical mycology, 2017, 55. Jg., Nr. 3, S. 334-343). Auch in vivo Versuche an Ratten zeigten ein ernstzunehmendes Schädigungspotential (Interdisciplinary Toxicology, 2015, 8. Jg., Nr. 4, S. 193-202).

Aus der EP 3 452 118 B1 ist eine Polyelektrolytbeschichtung bekannt, die mindestens eine polykationische Schicht, bestehend aus Polypeptiden, und mindestens eine polyanionische Schicht, bestehend aus Hyaluronsäure, umfasst. Die Polyelektrolytbeschichtung weist eine biozide Aktivität auf und kann zur Herstellung antimikrobieller medizinischer Vorrichtungen, wie beispielsweise Implantate, verwendet werden.

Der vorliegenden Erfindung liegt die Aufgabe zugrunde, eine Wundauflage zur Wundbehandlung im feuchten oder feucht-nassen Milieu der eingangs beschriebenen Art mit verbesserten antimikrobiellen Eigenschaften bereitzustellen. Zudem soll die Wundauflage atraumatisch von einer Wunde entfernbar sein und eine hohe Biokompatibilität aufweisen.

Diese Aufgabe wird durch eine Wundauflage mit einem faservliesbasierten Saug-/Spülkörper und mit einer die äußeren Sichtseiten der Wundauflage bildenden Umhüllung gelöst, wobei die Umhüllung auf der wundzugewandten Seite der Wundauflage ein textiles Flächenmaterial, insbesondere aus einem Gestrick, Gewirke oder Gewebe, umfasst, und wobei die Umhüllung auf der wundzugewandten Seite der Wundauflage eine außenseitig teilweise oder vollständig aufgebrachte antimikrobielle Beschichtung aufweist, welche eine Hyaluronsäure sowie ein Polypeptid ausgewählt aus Polyarginin, Polylysin und Polyornithin oder eine Mischung aus wenigstens zwei der zuvor genannten Polypeptide umfasst. In dem Saug-/Spülkörper ist superabsorbierendes Material verteilt aufgenommen, wobei der Saug-/Spülkörper herstellerseitig mit einer salzhaltigen wässrigen Lösung, insbesondere Ringerlösung beaufschlagt ist.

Dadurch, dass die antimikrobielle Beschichtung auf der Umhüllung auf der wundzugewandten Seite der Wundauflage außenseitig aufgebracht ist, kann die von der Hyaluronsäure und den Polypeptiden ausgehende antimikrobielle Aktivität direkt an der Wundoberfläche genutzt werden. Somit können Mikroorganismen und Biofilme zerstört werden, da die antimikrobielle Beschichtung überwiegend im direkten Kontakt mit den Mikroorganismen wirkt. Die antimikrobielle Aktivität von Polyelektrolytbeschichtungen bestehend aus Polypeptiden und Hyaluronsäure ist in der EP 3 452 118 B1 beschrieben.

Es wurde festgestellt, dass bei Kontakt der antimikrobiellen Beschichtung mit dem superabsorbierenden Material im Saug-/Spülkörper die Polypeptide mit dem üblicherweise negativ geladenen superabsorbierenden Material interagieren und dadurch die antimikrobielle Aktivität gemindert wird. Somit ist erfindungsgemäß die antimikrobielle Beschichtung außenseitig auf der Wundauflage aufgebracht. Die antimikrobielle Beschichtung befindet sich auf dem textilen Flächenmaterial der Umhüllung und hat keinen direkten Kontakt zu dem Saug-/Spülkörper bzw. dem superabsorbierenden Material.

Dadurch, dass die erfindungsgemäße antimikrobielle Beschichtung aufgrund der Hyaluronsäure eine gelartige Struktur ausbildet, kann sich das textile Flächenmaterial nicht mit der Wunde oder Wundbestandteilen verbinden. Weder kann Gewebe in das textile Flächenmaterial einwachsen, noch kann das textile Flächenmaterial und somit die Wundauflage am Wundbett anhaften. Somit lässt sich die Wundauflage atraumatisch von der Wunde entfernen. Der für gewöhnlich notwendige Einsatz von Salbe oder Silikongel zum Erreichen der atraumatischen Eigenschaften ist bei der vorliegenden Erfindung nicht notwendig, da diese Rolle von der antimikrobiellen Beschichtung erfüllt wird.

Die erfindungsgemäße Wundauflage eignet sich auch zur Behandlung von Wunden mit antibiotikaresistenten Bakterien. Während Antibiotika üblicherweise organische Verbindungen sind, die von resistenten Bakterien beispielsweise abgebaut werden, ist ein derartiges Auftreten von Resistenzen im Hinblick auf die antimikrobielle Beschichtung der vorliegenden Erfindung nicht beobachtet worden.

Zudem weist die erfindungsgemäße Wundauflage weitere wundheilungsfördernde Eigenschaften auf, die sich durch die feuchtigkeitsregulierenden Eigenschaften der Hyaluronsäure ergeben. Solche Effekte sind in vivo im Bereich der extrazellulären Matrix zu finden.

Überraschenderweise hat sich gezeigt, dass die antimikrobielle Beschichtung durchleitend für Wundexsudat ist. Dies ermöglicht, dass die antimikrobielle Beschichtung vollflächig oder nahezu vollflächig auf das textile Flächenmaterial aufgebracht sein kann. Die antimikrobielle Beschichtung kann jedoch auch nur teilweise aufgebracht sein. Dies bedeutet, dass die antimikrobielle Beschichtung nur gewisse Abschnitte des textilen Flächenmaterials bedeckt oder beispielsweise nur an Fäden oder Fasern des textilen Flächenmaterials anhaftet, so dass die Durchgänge und Öffnungen zwischen den Fäden oder Fasern frei von der antimikrobiellen Beschichtung sind.

Eine Beschichtung liegt erfindungsgemäß auch dann vor, wenn die Beschichtung nicht geschlossen oder vollflächig auf dem textilen Flächenmaterial vorliegt. Selbst wenn nur einzelne Fäden oder Fasern die antimikrobielle Beschichtung aufweisen, wird erfindungsgemäß von der antimikrobiellen Beschichtung gesprochen.

Erfindungsgemäß umfasst die Umhüllung auf der wundzugewandten Seite der Wundauflage ein textiles Flächenmaterial, insbesondere aus einem Gestrick, Gewirke oder Gewebe. Diese Materialien ermöglichen aufgrund der Durchgänge und Öffnungen zwischen den Fäden und Maschen einen Flüssigkeitsaustausch zwischen Wunde und dem Saug-/Spülkörper. Wundsekrete werden durch den Saug-/Spülkörper aktiv aufgenommen, wobei der Saug-/Spülkörper im Gegenzug die salzhaltige wässrige Lösung an die Wunde abgibt. Dadurch, dass das textile Flächenmaterial mit der antimikrobiellen Beschichtung versehen ist, werden Keime nicht nur auf der Wundoberfläche abgetötet, sondern auch eine Rückkontamination durch Keime, die aus dem Saug-/Spülkörper herausgespült werden, verhindert.

Zudem weist insbesondere ein Gewirke den Vorteil auf, dass es der gesamten Wundauflage eine hohe Flexibilität verleiht.

Hyaluronsäure und Polypeptide wie Polyarginin, Polylysin und Polyornithin sind Substanzen, die im menschlichen Körper vorkommen und von diesem selbst produziert werden. Dadurch, dass die antimikrobielle Beschichtung diese körpereigenen Substanzen umfasst, weist die erfindungsgemäße Wundauflage eine hohe Biokompatibilität auf.

Bei dem im Rahmen der Erfindung eingesetzten Polypeptiden handelt es sich um Polyarginin und/oder Polylysin und/oder Polyornithin. Bei Polylysin kann es sich um α-Poly-L-Lysin und/oder ε-Poly-L-Lysin handeln. Das Polylysin kann beispielsweise eine Molekülmasse von 3,5 kDa bis 4 kDa aufweisen. Weiterhin kann das Polylysin pro Molekül 11 bis 50 Untereinheiten, bevorzugt 15 bis 35 Untereinheiten und besonders bevorzugt 20 bis 30 Untereinheiten Lysin umfassen. Ebenso kann das Polyarginin pro Molekül 11 bis 50 Untereinheiten, bevorzugt 15 bis 35 Untereinheiten und besonders bevorzugt 20 bis 30 Untereinheiten Arginin umfassen.

Alle diese genannten Polypeptide tragen aufgrund ihrer chemischen Eigenschaften eine positive Nettoladung und können somit antimikrobiell wirken. Polyarginin hat darüber hinaus zusätzlich den Vorteil die Wundheilung zu fördern, indem der Anteil sogenannter M2 Makrophagen innerhalb der Makrophagen-Population erhöht wird. Während Makrophagen vom Typ M1 Entzündungsreaktionen initiieren und die Produktion von cytotoxischen Radikalen einleiten, wirken M2 Makrophagen entzündungshemmend, proliferativ und fördern den Gewebeverschluss.

Bevorzugt enthält die erfindungsgemäße Beschichtung Polyarginin. Der Einsatz derartiger erfindungsgemäßer polyargininhaltiger Beschichtungen ist selbst bei nicht infizierten Wunden vorteilhaft, da die Wundheilung beschleunigt wird.

Alternativ ist die Kombination aus Polyarginin und Polylysin besonders bevorzugt, da diese in der Beschichtung eine besonders ausgeprägte antimikrobielle Wirkung aufweist, die vermutlich auf einen synergistischen Effekt zurückzuführen ist. Die Kombination aus Polyarginin und Polylysin vermag die antimikrobielle Wirkung der jeweiligen Einzelsubstanz gegenüber diversen Erregern zu übertreffen.

Die Polypeptide können eine im Wesentlichen einzige Kettenlänge aufweisen. Eine im Wesentlichen einzige Kettenlänge liegt dann vor, wenn mindestens 90 %, vorzugsweise mindestens 95 %, besonders bevorzugt mindestens 98 % und insbesondere mindestens 99 % aller in der antimikrobiellen Beschichtung enthaltenen Polypeptide dieselbe Kettenlänge haben. Dies bietet den Vorteil, dass die antimikrobielle Wirkung und die Stabilität der antimikrobiellen Beschichtung einfach kontrollierbar ist.

Bevorzugt liegt die Anzahl der Aminosäuren in den Polypeptiden bei wenigstens 10. Weiterhin liegt die Anzahl der Aminosäuren in den Polypeptiden bevorzugt bei höchstens 2000. Besonders bevorzugt ist eine Anzahl von 10 bis 100 Aminosäuren und insbesondere 30 bis 50 Aminosäuren. Diese Anzahl bezieht sich auf die Aminosäuren pro Molekül eines Polypeptids. Typischerweise sind diese Aminosäuren über Peptidbindungen miteinander verbunden.

Gemäß einer bevorzugten Ausführungsform der Erfindung weisen die Polypeptide eine Molekülmasse von 1 bis 41 kDa auf. Bevorzugt haben die Polypeptide eine Molekülmasse von 2 bis 40 kDa, besonders bevorzugt 3 bis 39 kDa und insbesondere bevorzugt 5 bis 37 kDa.

Hyaluronsäure, auch bekannt als Hyaluron, ist ein zu den Glycosaminoglycanen zählendes Heteropolysaccharid. Grundbaustein von Hyaluronsäure ist ein aus d-Glucuronsäure und N-Acetyl-d-glucosamin alternierendes in (1→3)-(1→4)-β-glycosidischer Bindung aufgebautes Aminodisaccharid. Hyaluronsäuren tragen aufgrund ihrer chemischen Eigenschaften eine negative Nettoladung und gehören somit zu den Polyanionen. Hyaluronsäure ist wasserbindend und hat geweberegenerierende und wundheilende Eigenschaften.

Vorzugsweise kann die Hyaluronsäure im Rahmen dieser Erfindung Molmassen von ca. 50 bis ca. 10⁴ kg/mol aufweisen, bevorzugt wird Hyaluronsäure mit einer molaren Masse von 140 bis 150 kg/mol verwendet. Im Rahmen dieser Erfindung ist es auch möglich, eine Mischung von Hyaluronsäuremolekülen unterschiedlicher Molmassen zu verwenden, wobei in diesem Fall die Molmasse als durchschnittliche Molmasse aller Hyaluronsäuremoleküle in der Mischung angegeben werden kann. Beispielsweise kann die durchschnittliche Molmasse 143 bis 146 kg/mol betragen. Alternativ haben alle oder im Wesentlichen alle Hyaluronsäuremoleküle in der Beschichtung dieselbe Molmasse.

Die Hyaluronsäure kann als Polymermischung von Polymeren der Hyaluronsäure mit unterschiedlicher Kettenlänge vorliegen. Die Polymere der Hyaluronsäure in der Polymermischung können ein Molekulargewicht von wenigstens 10 kDa und höchstens 300 kDa aufweisen.

Die Hyaluronsäure kann quervernetzt oder unvernetzt vorliegen, wobei unvernetzte Hyaluronsäure bevorzugt ist. Mögliche Verfahren zur Quervernetzung von Hyaluronsäuren sind der Einsatz von 1,4-Butanediol Diglycidyl Ether (BDDE), enzymatische Quervernetzung (z.B. mittels Transglutaminasen) oder physikalische Quervernetzung (z.B. mittels Gefrieren, Erhitzen, Ultraschall, Mikrowellen). Ein mögliches Verfahren zur Quervernetzung von Hyaluronsäuren wird in der WO 2010/131175 A1 beschrieben.

Ein weiterer Vorteil der Hyaluronsäure in der antimikrobiellen Beschichtung besteht darin, dass durch die elektrostatischen und ionischen Wechselwirkungen zwischen negativ geladener Hyaluronsäure und positiv geladenen Polypeptiden ein stabiler Verbund aus Hyaluronsäure und Polypeptiden in der antimikrobiellen Beschichtung ermöglicht wird. Die Hyaluronsäure kann somit als Anker für die Polypeptide dienen. Selbstverständlich ist dies auch umgekehrt möglich, nämlich dass die Polypeptide die Hyaluronsäure verankern. Beispielsweise kann bei einem positiv geladenen textilen Flächenmaterial somit mittels der Hyaluronsäure auch die antimikrobielle Beschichtung, die ja auch die positiv geladenen Polypeptide umfasst, stabil an das textile Flächenmaterial gebunden werden.

Die antimikrobielle Beschichtung kann weitere Verbindungen oder strukturelle Komponenten umfassen. Diese können positiv geladen, negativ geladen oder ladungsneutral sein.

Gemäß einer Ausgestaltung der Erfindung kann eine wässrige Pufferlösung in der antimikrobiellen Beschichtung vorgesehen sein. Beispiele für wässrige Pufferlösungen sind Citratpuffer, Ringerlösung, TRIS-Puffer, TE-Puffer, TBS-Puffer und TBS-T-Puffer. Bevorzugt handelt es sich bei dem Puffer um Tris-NaCl-Puffer. Die Konzentration des Puffers im Lösungsmittel (z. B. Wasser) kann beispielsweise 5 mmol bis 300 mmol betragen, bevorzugt beträgt die Konzentration 10 mmol bis 200 mmol.

In einer weiteren bevorzugten Ausführungsform kann die antimikrobielle Beschichtung einen Konservierungsstoff oder einen Stabilisator in einer Menge von 0,1 bis 2 Gew.-% enthalten. Beispiele für geeignete Konservierungsstoffe sind Benzoesäure, Sorbinsäure oder Parabene. Beispiele für geeignete Stabilisatoren sind Ascorbylpalmitat und Tocopherol.

Der Gehalt an polaren Flüssigkeiten, insbesondere an Wasser, in der antimikrobiellen Beschichtung kann 0,1 bis 50 Gew.-% betragen. In manchen Fällen, beispielsweise wenn die Beschichtung als Gel vorliegen soll, können auch mehr als 50 Gew.-% an polaren Flüssigkeiten gewünscht und sinnvoll sein. Bevorzugt enthält die Beschichtung 1 bis 45 Gew.-% polare Flüssigkeiten, besser 3 bis 40 Gew.-% polare Flüssigkeiten und am besten enthält die Beschichtung 5 bis 35 Gew.-% polare Flüssigkeiten. Diese Konzentrationen beziehen sich auf den finalen Gehalt in der antimikrobiellen Beschichtung nach aktiver oder passiver Trocknung.

Vorzugsweise kann die antimikrobielle Beschichtung eine Schichtdicke von 10 nm bis 1000 nm aufweisen. Weitere bevorzugte Schichtdicken liegen zwischen 50 nm und 900 nm, 100 nm und 800 nm und 200 nm bis 600 nm. Größere Schichtdicken führen dazu, dass die Wundauflage besonders ausgeprägte atraumatische Eigenschaften aufweist. Beschichtungen, die eine geringe Dicke aufweisen, ermöglichen einen schnelleren Flüssigkeitsaustausch zwischen der Wunde und dem Saug-/Spülkörper.

Gemäß einer weiteren Ausführungsform können die Hyaluronsäure und das Polypeptid innerhalb der antimikrobiellen Beschichtung durchmischt vorliegen. Dies bedeutet, dass die Hyaluronsäure und das Polypeptid innerhalb der antimikrobiellen Beschichtung nahezu homogen verteilt sind.

Das Polypeptid kann alternativ auch in eine Hyaluronsäurematrix eingebettet sein.

Die antimikrobielle Beschichtung umfasst in einer Ausführungsform wenigstens eine Polypeptidlage, die die Polypeptide umfasst und eine positive Nettoladung aufweist, und wenigstens eine Hyaluronsäurelage, die die Hyaluronsäure umfasst und eine negative Nettoladung aufweist. Die wenigstens eine Polypeptidlage und die wenigstens eine Hyaluronsäurelage liegen übereinander und sind miteinander verbunden, wobei sich jeweils die wenigstens eine Polypeptidlage mit der wenigstens einen Hyaluronsäurelage abwechselt, so dass eine Abfolge von alternierenden Lagen gebildet ist.

Die Anzahl der alternierenden Lagen kann dabei gerade oder ungerade sein. Eine gerade Anzahl an alternierenden Lagen ist dabei bevorzugt, weil dabei für jede negativ geladene Lage eine positiv geladene Lage zur Verfügung steht und sich die gegensätzlichen Ladungen anziehen, was zu einer besonders stabilen Beschichtung führt.

Bevorzugt bilden je eine Hyaluronsäurelage und eine Polypeptidlage zusammen eine Doppelschicht aus.

Vorzugsweise beträgt die Anzahl der alternierenden Lagen 20 bis 100, bevorzugt 30 bis 90, besonders bevorzugt 40 bis 80 und ganz besonders bevorzugt 50 bis 70. Dabei besteht die eine Hälfte der Lagen aus Hyaluronsäurelagen und die andere Hälfte der Lagen aus Polypeptidlagen.

Des Weiteren ist bevorzugt, dass die Polypeptide in eine Hyaluronsäurematrix eingebettet sind. Diese kann dadurch gebildet werden, dass eine ungerade Anzahl an alternierenden Lagen vorliegt und die jeweils äußere Lage eine Hyaluronsäurelage ist.

Es erweist sich als vorteilhaft, wenn der faservliesbasierte Saug-/Spülkörper cellulosische Fasern, insbesondere eine Mischung aus cellulosischen Fasern und thermoplastischen Fasern, insbesondere Polyolefin-Fasern, insbesondere Polypropylen-Fasern oder Polypropylen/Polyethylen-Fasern, umfasst.

Dabei beträgt das Flächengewicht des Faseranteils des Saug-/Spülkörpers vorteilhafterweise 20 bis 500 g/m², bevorzugt 30-300 g/m², besonders bevorzugt 50-200 g/m².

Weiter erweist es sich als vorteilhaft, wenn das textile Flächenmaterial aus einem thermoplastischen Material, insbesondere aus Polyolefin, insbesondere aus Polypropylen, gebildet ist.

Gemäß einer besonders bevorzugten Ausführungsform der Erfindung weist die Umhüllung auf der wundzugewandten Seite eine zusätzliche außenseitig partiell und strukturiert aufgebrachte atraumatisch wirkende Schicht aus Silikon mit einem Bedeckungsgrad von höchstens 70 % auf. Somit kann das atraumatische Entfernen der Wundauflage auch nach längerer Tragezeit sichergestellt werden, sollte die Hyaluronsäure vom Körper resorbiert worden sein.

Weiter erweist es sich als vorteilhaft, wenn der Bedeckungsgrad der partiell und strukturiert aufgebrachten atraumatisch wirkenden Beschichtung 20 - 70 %, insbesondere 25 - 50 %, insbesondere 30 - 40 % beträgt.

Es erweist sich weiter als vorteilhaft, wenn die partiell und strukturiert aufgebrachte atraumatisch wirkende Beschichtung streifenförmig ausgebildet ist. Die Streifen können dabei linear erstreckt sein. Sie verlaufen vorzugsweise parallel oder äquidistant zueinander. Die Breite eines Streifens beträgt vorteilhafterweise 1 bis 3 mm. Der Abstand der Streifen voneinander beträgt vorteilhafterweise 4 bis 8 mm, insbesondere 4 bis 6 mm. Dadurch wird der Flüssigkeitsaustausch zwischen Wunde und Saug-/Spülkörper nicht wesentlich beeinträchtigt.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden.

Es zeigen:
- Figur 1: eine schematische Schnittansicht einer beispielhaften erfindungsgemäßen Wundauflage,
- Figur 2: eine fluoreszenzmikroskopische Aufnahme eines beispielhaften textilen Flächenmaterials mit antimikrobieller Beschichtung,
- Figur 3: eine fluoreszenzmikroskopische Aufnahme eines beispielhaften textilen Flächenmaterials ohne antimikrobielle Beschichtung,
- Figur 4: ein Diagramm der Werte für die Aktivität einer beispielhaften antimikrobiellen Beschichtung umfassend Polyarginin und
- Figur 5: ein Diagramm der Werte für die Aktivität einer beispielhaften antimikrobiellen Beschichtung umfassend Polylysin.

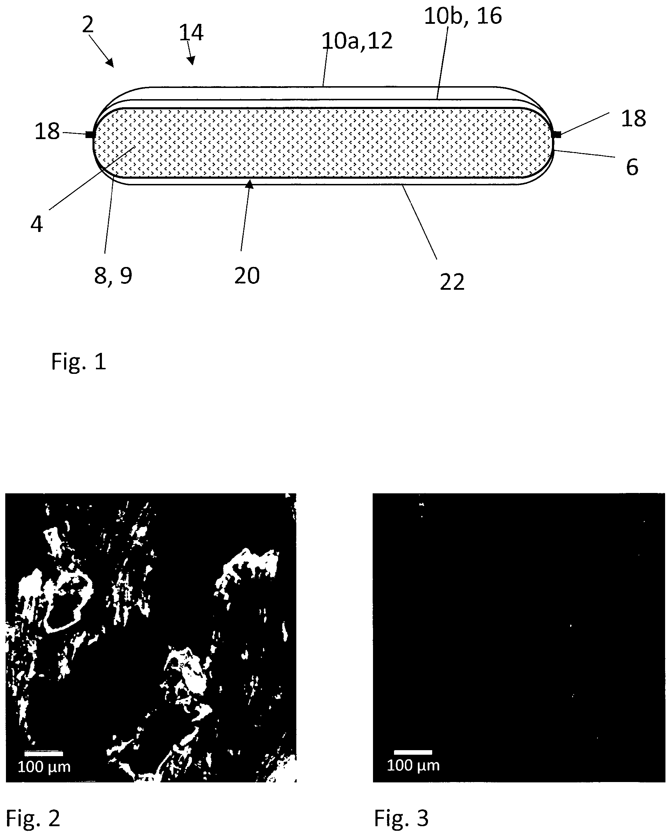

Figur 1 zeigt im Schnitt eine Wundauflage 2. Sie umfasst einen Saug-/Spülkörper 4 auf Faservliesbasis. Bei dieser Faserbasis handelt es sich um eine bevorzugte Mischung aus luftgelegten Zellulosefasern (Zellstoff) und Polypropylen-Fasern oder Polypropylen/Polyethylen-Fasern. Dieser Fasermischung sind superabsorbierende Polymermaterialien (SAP) in Partikelform oder in Faserform möglichst homogen beigemischt, wobei der SAP-Anteil an der Gesamtmasse des Saug-/Spülkörpers 4 vorzugsweise 40 - 50 Gew.-% beträgt. Die mittlere Korngröße der SAP-Partikel liegt beispielsweise bei 150 bis 850 µm (beispielsweise Polyacrylat der Marke Favor pac 300 von der Firma Evonik Stockhausen GmbH).

Der Saug-/Spülkörper 4 ist von einer die Außenseiten der Wundauflage bildenden Umhüllung 6 umgeben, die von einer wundzugewandten Hüllschicht 8 und zwei wundabgewandten Hüllschichten 10a, b gebildet ist. Bei der wundzugewandten Hüllschicht 8 handelt es sich vorzugsweise um eine Schicht 9 aus einem textilen Flächenmaterial, wie ein Gestrick, vorzugsweise aus Polypropylen, wobei auch ein Gewebe oder Gewirke in vorteilhafter Weise denkbar wäre, also eine Hüllschicht aus Fäden oder Filamenten mit textiler Bindung, die einen guten Flüssigkeitsaustausch zwischen dem Saug-/Spülkörper 4 und der Wundumgebung gestattet.

Die eine wundabgewandte Hüllschicht 10a ist eine Faservliesschicht 12, vorzugsweise aus Polypropylen, welche eine wundabgewandte Sichtseite 14 der Wundauflage 2 bildet. Die zweite Hüllschicht 10b ist von einer flüssigkeitsundurchlässigen Kunststofffilmschicht 16 gebildet, die unmittelbar unterhalb der Faservliesschicht 12, also auf der wundzugewandten Seite der Faservliesschicht 12, zwischen der Faservliesschicht 12 und dem Saug-/Spülkörper 4 angeordnet ist. Diese beiden wundabgewandten Hüllschichten 10a, 10b sind nicht flächenhaft miteinander verbunden, sie bilden also kein Laminat im eigentlichen Sinne, sondern sie liegen lose und verschieblich flächenhaft aneinander an, wobei sie aber entlang eines Umfangsrands 18 oder eines Umfangsrandbereichs miteinander und mit den weiteren Komponenten der Wundauflage verbunden sind.

Auf der wundzugewandten Außenseite 20 der wundzugewandten Hüllschicht 8 ist eine teilweise oder vollständig aufgebrachte antimikrobielle Beschichtung 22 vorgesehen. Diese Beschichtung 22 umfasst eine Hyaluronsäure sowie ein Polypeptid ausgewählt aus Polyarginin, Polylysin und Polyornithin oder eine Mischung aus wenigstens zwei der zuvor genannten Polypeptide. Durch den Kontakt mit der Wunde wirkt die Beschichtung 22 antimikrobiell und verhindert ein Einwachsen des Gewebes in die Wundauflage 2, so dass die Wundauflage 2 atraumatisch von der Wunde entfernt werden kann.

Die Wundauflage 2 ist mit salzhaltiger wässriger Lösung beaufschlagt. Diese befindet sich im Saug-/Spülkörper 4. Dieser salzhaltigen Lösung kann eine Substanz mit antimikrobieller Wirkung zugegeben sein, welche im feuchten oder feuchtnassen Wundmilieu bei pH-Werten im leicht sauren bis neutralen Bereich von pH 4 bis 7,5 kationisch vorliegt. Diese kationische Substanz mit antimikrobieller Wirkung wird von negativen Gruppen des anionischen superabsorbierenden Materials derart angezogen, dass sie auch im flüssigkeitsaustauschenden Betrieb des Saug-/Spülkörpers 4 an die superabsorbierenden Materialien gebunden bleibt, also weitestgehend nicht in das Wundmilieu abgegeben wird. Hierdurch werden mit Wundsekret in den Saug-/Spülkörper 4 eingebrachte Keime an einer Vermehrung gehindert, wodurch eine Rücckontamination in Richtung auf die Wunde weitestgehend verhindert wird.

Bei einer beispielhaften bevorzugten Zusammensetzung der Wundauflage 2 besteht die Faservliesbasis des Saug-/Spülkörpers 4 aus 33 g/m² Zellulosefasern (Zellstoff) und 11 g/m² Polypropylen/Polyethylen-Fasern als Bindefasern. Dieser Fasermischung sind 70 g/m² der oben genannten superabsorbierenden Polymermaterialien (SAP) homogen beigemischt. Die Wundauflage 2 ist mit so viel Ringerlösung aktiviert bzw. beaufschlagt, dass im Wesentlichen eine Sättigung des Saug-/Spülkörpers 4 mit Ringerlösung erreicht ist. Die Umhüllung 6 ist wie vorausgehend beschrieben ausgebildet.

Die antimikrobielle Beschichtung 22 kann entweder mittels einem Sprühverfahren oder schichtweise mittels einem Tauchverfahren auf das textile Flächenmaterial 9, bei dem es sich um ein Gewirke aus Polypropylen handelt, aufgebracht sein. Hierzu wurden folgende Lösungen bereitgestellt:

**Tabelle 1**

| | Polymer | MW (kDa) | Hersteller | Konz. (mg/ml) |
|---|---|---|---|---|
| PAR30 | Poly(L-Arginin), 30 Arginin-Einheiten | 5, 8 | Alamanda Polymers | 0,5 |
| ε-PLL | ε-Poly(L-Lysin) | 3,5 - 4,5 | BIOSYNTH | 10 |
| HA144 | Hyaluronsäure | 144 | Lifecore Biomedical | 0,5 |
| HA119 | Hyaluronsäure | 119 | Lifecore Biomedical | 0,5 |

| | | | | |
|---|---|---|---|---|
| MW = Molekülmasse Konz. = Konzentration, eingestellt mit Tris-NaCl-Puffer umfassend 150 mM NaCl, 10 mM tris(hydroxymethyl)-aminomethan (TRIS, Merck, Deutschland), pH 7,4. | | | | |

### Beispiel 1:

Das textile Flächenmaterial 9, nämlich ein Gewirke aus Polypropylen, wurde abwechselnd in die Lösungen "PAR30", um eine Polypeptidlage aus Polyarginin aufzutragen, und HA119, um eine Hyaluronsäurelage aufzutragen, getaucht. Nach jedem Tauchschritt wurde das textile Flächenmaterial 9 mit Tris-NaCl-Puffer (150 mM NaCl, 10 mM tris(hydroxymethyl)-aminomethan, pH 7,4) gespült. Insgesamt wurde das textile Flächenmaterial 9 24 mal in jede Lösung getaucht, so dass eine Abfolge von 48 alternierenden Lagen gebildet wurde bzw. 24 Doppellagen. Anschließend wurde das textile Flächenmaterial 9 mit der antimikrobiellen Schicht 22 bestehend aus 24 Doppellagen Polyarginin/Hyaluronsäure über Nacht passiv bei Raumtemperatur getrocknet.

Das in der so erzeugten antimikrobiellen Beschichtung 22 enthaltene Polyarginin wurde mit Fluoresceinisothiocyanat (FITC) markiert und unter einem Konfokalmikroskop sichtbar gemacht.

Figur 2 zeigt eine fluoreszenzmikroskopische Aufnahme des mit der antimikrobiellen Beschichtung 22 versehenen textilen Flächenmaterials 9 gemäß Beispiel 1. Die fluoreszierenden Bereiche sind weiß dargestellt. Die antimikrobielle Beschichtung 22 haftet auf der Faserstruktur des Gewirkes, so dass auf eine stabile Beschichtung des textilen Flächenmaterials 9 geschlossen werden kann.

Figur 3 zeigt eine fluoreszenzmikroskopische Aufnahme des unbeschichteten textilen Flächenmaterials 9 als Kontrolle. Es sind keine weißen fluoreszierende Bereiche erkennbar.

Antimikrobielle Evaluierung:
Die erfindungsgemäßen Wundauflagen 2 wurden zur Evaluierung der antimikrobiellen Wirksamkeit einem Test auf antimikrobielle Aktivität nach ISO 20743:2021 unterzogen (Owen L, Laird K. Development of a silver-based dual-function antimicrobial laundry additive and textile coating for the decontamination of healthcare laundry. J Appl Microbiol. 2021; 130 (4) :1012-22) . Nach 24 Stunden Kontaktzeit der Wundauflagen mit einer gramnegativen Kultur von Pseudomonas aeruginosa (ATTC 27853) und/oder einer grampositiven Kultur von Staphylococcus aureus (ATTC 25923) wurde die Anzahl teilungsfähiger Bakterienzellen (CFU) auf dem textilen Flächenmaterial 9 im Vergleich zu einer Kontrolle ohne antimikrobielle Beschichtung bestimmt.

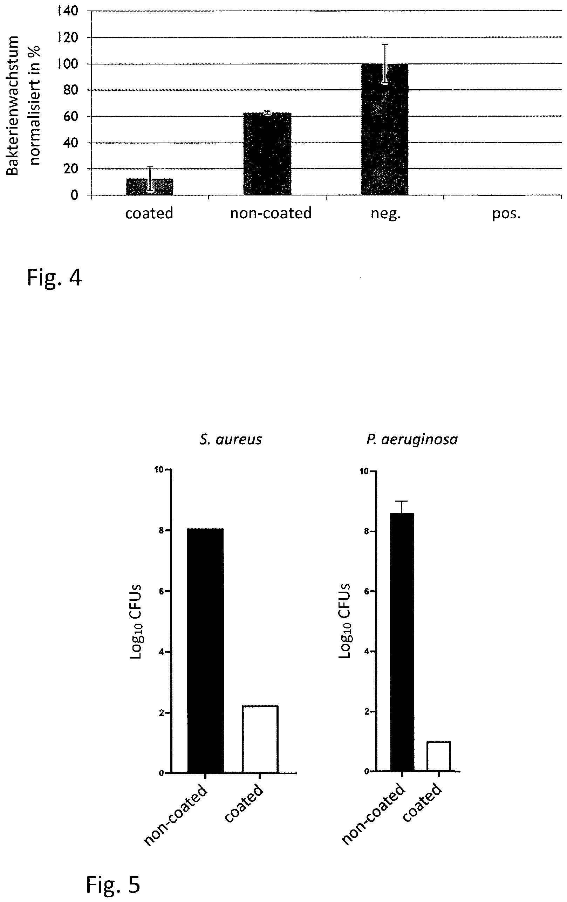

Figur 4 zeigt die Ergebnisse des Tests auf antimikrobielle Aktivität für Wundauflagen 2 gemäß Beispiel 1, bei denen das textile Flächenmaterial 9 mit 24 Doppellagen PAR30/HA119 beschichtet wurde ("coated") im Vergleich zu einer unbeschichteten Wundauflage ("non-coated"). Als Negativkontrolle ("neg.") diente eine Lösung aus *S. aureus,* als Positivkontrolle ("pos.") wurde dem Testsubstrat ein gegen *S. aureus* wirksames Antibiotikum zugesetzt. Die gegen die Negativkontrolle normalisierte Bakterienwachstumsrate konnte durch die antimikrobielle Beschichtung ("coated") auf ca. 10 % gesenkt werden. Die unbeschichteten Wundauflagen reduzierten das Bakterienwachstum lediglich um ca. 40 %. Durch Zugabe des Antibiotikums (pos.) konnte das Wachstum völlig gehemmt werden.

### Beispiel 2:

Das textile Flächenmaterial 9 der Wundauflage 2 wurde mittels einem Sprühverfahren mit ε-PLL und HA144 gemäß Tabelle 1 beschichtet. Beide Lösungen wurden zeitgleich über separate Düsen einer Sprühpistole auf das textile Flächenmaterial 9 aufgesprüht, so dass eine gemischte Lage der antimikrobiellen Beschichtung 22, bei der Hyaluronsäure und Polylysin statistisch verteilt vorliegen, erzeugt wurde. Insgesamt wurden zehn gemischte Lagen nacheinander überlagernd aufgetragen und anschließend über Nacht, passiv bei Raumtemperatur, getrocknet.

Figur 5 zeigt die Ergebnisse des Tests auf antimikrobielle Aktivität für Wundauflagen 2, die gemäß Beispiel 2 mit dem Sprühverfahren beschichtet wurden. Mit "non-coated" sind Wundauflagen ohne antimikrobielle Beschichtung 22 bezeichnet. Die mit dem Sprühverfahren beschichteten erfindungsgemäßen Wundauflagen 2 sind mit "coated" bezeichnet. Durch die antimikrobielle Beschichtung 22 mit Polylysin (ε-PLL) und HA144 konnte nach 24 Stunden sowohl bei *S. aureus* als auch bei *P. aeruginosa* eine Reduktion der CFU über mehr als fünf Logstufen verglichen mit der unbeschichteten Kontrolle ("non-coated") erreicht werden.

## Patentansprüche

1. Wundauflage (2) zur Wundbehandlung im feuchten oder feuchtnassen Milieu, mit einem faservliesbasierten Saug-/Spülkörper (4), in dem superabsorbierendes Material verteilt aufgenommen ist, wobei der Saug-/Spülkörper (4) herstellerseitig mit einer salzhaltigen wässrigen Lösung, insbesondere Ringerlösung beaufschlagt ist, und mit einer die äußeren Sichtseiten der Wundauflage bildenden Umhüllung (6), wobei die Umhüllung (6) auf der wundzugewandten Seite der Wundauflage ein textiles Flächenmaterial (9), insbesondere aus einem Gestrick, Gewirke oder Gewebe, umfasst, **dadurch gekennzeichnet, dass** die Umhüllung (6) auf der wundzugewandten Seite der Wundauflage eine außenseitig teilweise oder vollständig aufgebrachte antimikrobielle Beschichtung (22) aufweist, welche eine Hyaluronsäure sowie ein Polypeptid ausgewählt aus Polyarginin, Polylysin und Polyornithin oder eine Mischung aus wenigstens zwei der zuvor genannten Polypeptide umfasst.

2. Wundauflage (2) nach Anspruch 1, **dadurch gekennzeichnet, dass** die Polypeptide eine Anzahl von 10 bis 100 Aminosäuren, insbesondere 30 bis 50 Aminosäuren aufweisen.

3. Wundauflage (2) nach Anspruch 1 oder 2, **dadurch gekennzeichnet, dass** die Hyaluronsäure als Polymermischung von Polymeren der Hyaluronsäure mit unterschiedlicher Kettenlänge vorliegt und die Polymermischung Polymere der Hyaluronsäure mit einem Molekulargewicht von mindestens 10 kDa und höchstens 300 kDa umfasst.

4. Wundauflage (2) nach einem der vorhergehenden Ansprüche, **dadurch gekennzeichnet, dass** die Polypeptide ein Molekulargewicht von 1 bis 41 kDa haben.

5. Wundauflage (2) nach einem der vorhergehenden Ansprüche, **dadurch gekennzeichnet, dass** das Polypeptid und die Hyaluronsäure innerhalb der antimikrobiellen Beschichtung (22) durchmischt vorliegen.

6. Wundauflage (2) nach einem der Ansprüche 1 bis 4, **dadurch gekennzeichnet, dass** die antimikrobielle Beschichtung (22) wenigstens eine Polypeptidlage, die das Polypeptid umfasst und eine positive Nettoladung aufweist, und wenigstens eine Hyaluronsäurelage, die die Hyaluronsäure umfasst und eine negative Nettoladung aufweist, umfasst, wobei die wenigstens eine Polypeptidlage und die wenigstens eine Hyaluronsäurelage übereinanderliegen und miteinander verbunden sind, wobei sich jeweils die wenigstens eine Polypeptidlage mit der wenigstens einen Hyaluronsäurelage abwechselt, so dass eine Abfolge von alternierenden Lagen gebildet ist.

7. Wundauflage (2) nach Anspruch 6, **dadurch gekennzeichnet, dass** die Anzahl der wenigstens einen Polypeptidlage und der wenigstens einen Hyaluronsäurelage in der antimikrobiellen Beschichtung (22) 10 bis 100 beträgt.

8. Wundauflage (2) nach einem der vorhergehenden Ansprüche, **dadurch gekennzeichnet, dass** die antimikrobielle Beschichtung (22) eine Dicke von 10 nm bis 1000 nm aufweist.

9. Wundauflage (2) nach einem der vorhergehenden Ansprüche, **dadurch gekennzeichnet, dass** der faservliesbasierte Saug-/Spülkörper (4) cellulosische Fasern, insbesondere eine Mischung aus cellulosischen Fasern und thermoplastischen Fasern, insbesondere Polyolefin-Fasern, insbesondere Polypropylen-Fasern oder Polypropylen/Polyethylen-Fasern, umfasst.

10. Wundauflage (2) nach einem der vorhergehenden Ansprüche, **dadurch gekennzeichnet, dass** das Flächengewicht des Faseranteils des Saug/Spülkörpers (4) 20-500g/m² beträgt.

11. Wundauflage (2) nach einem der vorhergehenden Ansprüche, **dadurch gekennzeichnet, dass** das textile Flächenmaterial (9) aus einem thermoplastischen Material, insbesondere aus Polyolefin oder Polypropylen, gebildet ist.

12. Wundauflage (2) nach einem der vorhergehenden Ansprüche, **dadurch gekennzeichnet, dass** das textile Flächenmaterial (9) eine positive Nettoladung aufweist.

13. Wundauflage (2) nach einem der vorhergehenden Ansprüche, **dadurch gekennzeichnet, dass** die Umhüllung (6) auf der wundzugewandten Seite des Weiteren eine außenseitig partiell und strukturiert aufgebrachte atraumatisch wirkende Schicht aus Silikon mit einem Bedeckungsgrad von höchstens 70 % aufweist.

14. Wundauflage (2) nach Anspruch 13, **dadurch gekennzeichnet, dass** der Bedeckungsgrad der partiell und strukturiert aufgebrachten atraumatisch wirkenden Schicht aus Silikon 20 - 70 %, insbesondere 25 - 50 %, insbesondere 30 - 40 % beträgt.

15. Wundauflage (2) nach Anspruch 13 oder 14, **dadurch gekennzeichnet, dass** die partiell und strukturiert aufgebrachte atraumatisch wirkende Schicht aus Silikon streifenförmig ausgebildet ist.
